# FASCICULE DE BREVET EUROPEEN

(11) **EP 0 863 933 B1**
(45) Date de publication et mention de la délivrance du brevet: **20.06.2001**
(21) Numéro de dépôt: 96941085.1
(22) Date de dépôt: 29.11.1996
(51) Int. Cl.: C08G 63/66, C08G 63/00, A61K 47/34

(54) **NOUVEAUX HYDROGELS A BASE DE COPOLYMERES TRISEQUENCES, LEUR PREPARATION ET LEUR APPLICATION**
HYDROGELE AUF BASIS VON DREIBLOCKCOPOLYMERISATE, IHRE HERSTELLUNG UND IHRE VERWENDUNG
NOVEL HYDROGELS CONTAINING TRIBLOCK COPOLYMERS, AND PREPARATION AND USE THEREOF

(30) Priorité: 29.11.1995 FR 9514144
(43) Date de publication de la demande: 16.09.1998
(73) Titulaire: CENTRE NATIONAL DE LA RECHERCHE SCIENTIFIQUE (CNRS), 75016 Paris (FR)
(72) Inventeur: VERT, Michel, F-34170 Castelnau-le-Lez (FR); LI, Suming, F-34170 Castelnau-le-Lez (FR); RASHKOV, Iliya, 1303 Sofia (BG); ESPARTERO-SANCHEZ, José-Luis, E-41005 Séville (ES)
(74) Mandataire: Tonnellier, Jean-Claude
(86) Numéro de dépôt international: FR9601901
(87) Numéro de publication internationale: WO9719973

(56) Documents cités:
- EP-A- 0 092 918
- WO-A-90/03768
- PATENT ABSTRACTS OF JAPAN vol. 011, no. 365 (C-460), 27 Novembre 1987 & JP 62 135504 A (MITSUI TOATSU CHEM INC), 18 Juin 1987,

## Description

L'invention a pour objet de nouveaux hydrogels à base de copolymère triséquencé, leur préparation et leur application.

On sait que les hydrogels sont obtenus classiquement avec des matériaux solides qui gonflent dans l'eau en absorbant des volumes d'eau notables. Les hydrogels sont généralement constitués par des polymères formant dans l'eau un réseau tridimensionnel capable de retenir notamment des grosses molécules telles que des protéines. Les hydrogels peuvent être préparés à partir de polymères hydrophiles réticulés.

Les hydrogels ont été préconisés notamment comme supports pharmaceutiques implantables permettant la libération progressive de drogues, et en particulier de macromolécules telles que des protéines. Les hydrogels sont généralement non biodégradables et la drogue est libérée par un phénomène de diffusion.

Il a été proposé récemment de réaliser des hydrogels biodégradables à base de copolymères triséquencés poly(hydroxyacide)-poly (éthylèneglycol)-poly(hydroxyacide) en utilisant des copolymères solubles dans l'eau, à terminaisons acrylate, que l'on réticule *in situ* par photopolymérisation de solutions aqueuses du polymère ; voir A.S. Sawhney et al., Macromolecules 26, 581-587 (1993).

Le document EP-A-0092918 décrit des implants rigides constitués par des copolymères séquencés non réticulés, et contenant un polypeptide ayant une activité pharmacologique. Ces implants gonflent progressivement en absorbant de l'eau présente dans le milieu vivant où ils sont implantés.

On a maintenant découvert un nouveau procédé permettant, au départ de certains copolymères triséquencés, l'obtention très rapide d'hydrogels mous contenant un poids d'eau au moins égal au poids de copolymère triséquencé. Ces hydrogels mous sont facilement déformables et peuvent notamment transiter à travers une aiguille creuse ayant un diamètre interne de 2 mm, et en particulier à travers une aiguille creuse de 1 mm de diamètre interne.

Alors que les hydrogels étaient principalement utilisés jusqu'à présent pour le piégeage et la libération progressive de macromolécules hydrophiles, les hydrogels de la présente invention présentent la particularité de pouvoir retenir non seulement des macromolécules hydrophiles, mais aussi des substances hydrophobes.

L'invention a donc pour objet un hydrogel à base de copolymère triséquencé et d'eau, tel que défini dans la revendication 1.

L'invention concerne également l'obtention d'un produit obtenu par lyophilisation d'un hydrogel tel que défini ci-dessus. Un tel lyophilisat est capable d'absorber rapidement une forte proportion d'eau et de redonner un hydrogel mou gonflé d'eau, analogue à l'hydrogel dont il est issu.

Les rapports (m + n)/p qui conviennent peuvent être déterminés dans chaque cas par des expériences de routine, comme cela sera précisé ci-après. Généralement, les rapports (m + n)/p convenables sont des nombres compris entre 1 et 5 environ.

En particulier, le rapport (m + n)/p peut être choisi pour que ledit gel soit capable de retenir une quantité d'eau au moins égale à deux fois le poids de copolymère qu'il contient.

Le nombre p peut varier notamment de 10 à 120 et en particulier de 15 à 100.

Bien entendu, les blocs X et Y peuvent être semblables, c'est-à-dire composés des mêmes motifs répétitifs.

L'étude des copolymères analogues à ceux de formule
(I) montre que lorsque le rapport (m + n)/p est suffisamment faible, les copolymères sont solubles dans l'eau. Lorsque ledit rapport augmente, les copolymères correspondants ne sont que faiblement solubles dans l'eau, et donnent des solutions troubles dues vraisemblablement à la formation de micelles. Lorsque ledit rapport augmente encore, les polymères deviennent insolubles dans l'eau : mis au contact de l'eau, les polymères, sous forme de poudres, ne passent pas en solution et ne donnent pas lieu non plus à la formation de micelles, par exemple après 24 heures de contact avec l'eau à température ambiante, même si les copolymères peuvent gonfler légèrement au contact de l'eau.

Conformément à la découverte qui est à la base de l'invention, de tels copolymères insolubles dans l'eau peuvent donner des hydrogels à partir de leur solution dans un solvant organique miscible à l'eau, selon la méthode indiquée ci-dessus. Toutefois, lorsque la longueur des chaînons hydrophobes X et Y, pour un même bloc central G, augmente (autrement dit lorsque le rapport (m + n)/p augmente) au-delà d'une certaine valeur, les hydrogels ainsi obtenus renferment des quantités d'eau de plus en plus faibles. Il est donc facile de déterminer expérimentalement, pour chaque type de polymère de formule (I), la valeur du rapport (m + n)/p au-delà de laquelle les hydrogels ne sont plus capables de retenir une masse importante d'eau : en fait, avec de tels polymères en solution dans le solvant organique miscible à l'eau, on n'observe pas, par addition d'eau, la formation d'un hydrogel gonflé d'eau mais plutôt la formation d'un précipité du copolymère qui occupe un volume inférieur à celui de la solution organique de départ.

On peut donc déterminer la longueur optimale des chaînes X et Y par de simples expériences de routine.

Par exemple, dans le cas des copolymères poly(acide lactique)-poly(oxyde d'éthylène)-poly(acide lactique), on observe que lorsque le rapport (m + n)/p est inférieur à 0,2 environ, les copolymères sont solubles dans l'eau. Lorsque ledit rapport est compris entre 0,2 et 1 environ, les copolymères ne sont que faiblement solubles dans l'eau et donnent des solutions troubles. Lorsque ledit rapport est supérieur à 1 environ, les copolymères sont insolubles dans l'eau. Avec ces derniers copolymères, on peut obtenir, au départ d'une solution organique du copolymère, des hydrogels capables de retenir une quantité appréciable d'eau, lorsque le rapport (m + n)/p est compris entre 1 et 5, et en particulier entre 1 et 4 environ. On obtient des hydrogels particulièrement satisfaisants notamment lorsque ce rapport varie dans la gamme de 1,5 à 3 environ. Les nombres m et n sont donc tels que le rapport (m + n)/p est supérieur à 1 et inférieur à une valeur maximum au-delà de laquelle ledit copolymère n'est plus apte à former un hydrogel capable, dans les conditions indiquées ci-dessus, de retenir un poids d'eau au moins égal au poids dudit copolymère.

On donnera ci-après d'autres précisions à ce sujet en décrivant la préparation des hydrogels.

Les blocs polymères que représentent X et Y, dans la formule (I), sont des blocs polyesters linéaires hydrophobes. Ce sont notamment des polyesters aliphatiques.

On sait que les polyesters aliphatiques peuvent être obtenus :
a) soit par polycondensation d'un hydroxyacide sur lui-même, ou par polycondensation de plusieurs hydroxyacides,
b) soit par polymérisation par ouverture de cycle de lactones,
c) soit encore par polycondensation de diacides et de diols.

Parmi les polyesters dérivés d'hydroxyacides, on peut citer notamment ceux qui dérivent des monomères choisis parmi l'acide lactique, l'acide glycolique, les monoesters d'acide malique (par exemple des monoesters d'alkyle ou d'aralkyle, ou encore des monoesters résultant de la monoestérification de l'acide malique par un principe actif hydroxylé, notamment un principe actif hydrophobe ; voir par exemple les brevets US 4 320 753 et 4 265 247), les lactides (D-lactide, L-lactide et D,L-lactide), le glycolide, la paradioxanone, etc. Les blocs polymères représentés par X et Y peuvent également être des copolymères formés par lesdits monomères entre eux.

Parmi les polymères dérivés de diacides et de diols, on peut citer par exemple le poly(succinate d'éthylèneglycol).

Le bloc polymère représenté par G dans la formule (I) est un polymère hydrophile qui peut être choisi par exemple parmi les suivants : poly(éthylèneglycol) , polyvinyl pyrrolidone, polyacrylamide, etc.

Les copolymères séquencés de formule (I) sont connus ou peuvent être préparés de façon connue.

On peut effectuer la polymérisation conduisant à la formation des chaînons X et Y en présence du polymère G préformé et ayant des extrémités convenablement fonctionnalisées. Par exemple, on a déjà décrit l'obtention de copolymères séquencés à base de poly(hydroxyacides) et de polyéthylèneglycol ; voir notamment P. Cerrai et al., Journal of Materials Science : Materials in Medicine 5(1994) 308-313 et la demande de brevet EP-295 055. Les produits de départ sont d'une part le lactide et d'autre part le polyéthylèneglycol. On opère de préférence par polymérisation en masse en présence d'un catalyseur qui peut être un métal, un composé organo-métallique ou un acide de Lewis. Parmi les catalyseurs utilisés, on peut citer la poudre de zinc, l'hydrure de calcium, l'hydrure de sodium, l'octanoate d'étain, etc. La longueur des chaînes poly(hydroxyacide) dépend essentiellement du rapport molaire (motifs lactiques)/(PEG) dans le mélange initial. La longueur des chaînes poly(hydroxyacide) croît également avec la durée de la réaction qui peut aller par exemple de quelques minutes à quelques jours.

En utilisant des blocs polymères de type G aux extrémités fonctionnalisées, portant par exemple des groupes terminaux hydroxyle, acide carboxylique, amino, thiol, etc., il est possible d'obtenir des copolymères séquencés, répondant à la formule (I) ci-dessus, dans lesquels la jonction entre X et G et entre Y et G se fait par l'intermédiaire d'un groupement ester, amide, uréthanne, thioester, etc.

Les copolymères de formule (I) peuvent également être préparés à partir de blocs polymères préformés, en utilisant un polymère de type G dont les deux extrémités sont, de façon connue, convenablement fonctionnalisées, et des polymères de type X et Y ayant une seule extrémité fonctionnalisée de façon à pouvoir réagir avec une extrémité fonctionnalisée de G, avec établissement d'une liaison covalente.

Les hydrogels de l'invention forment un réseau tridimensionnel de chaînes hydrophiles (G) reliées par des microdomaines hydrophobes constituées par agrégation de chaînons poly(hydroxyacide) (X et Y). Il en résulte une réticulation physique des chaînes hydrophiles. Les microdomaines hydrophobes formés par les agrégats de chaînons X et Y sont analogues à des nanoparticules qui présentent la particularité de ne pas être libres et indépendantes, car elles font partie du réseau tridimensionnel formé par le copolymère dans l'hydrogel. Ces sortes de nanoparticules sont capables de solubiliser et donc de retenir des substances hydrophobes, par exemple des médicaments.

Les hydrogels de l'invention peuvent également retenir dans leur réseau tridimensionnel des macromolécules hydrosolubles, telles que des protéines, qui peuvent être par exemple, des médicaments.

Par exemple, on a incorporé dans des hydrogels selon l'invention d'autres protéines (notamment des immunoglobulines) et des conjugués protéine-antigène dont la protéine était l'anatoxine tétanique et les antigènes étaient soit des polyosides ou fragments de polyosides de *Streptococcus pneumoniae*, soit des polyosides ou fragments de polyosides de *Salmonella typhi.*

Les hydrogels de l'invention sont des gels évolutifs, progressivement dégradés par des réactions d'hydrolyse qui clivent, de façon aléatoire, des groupements esters des blocs X et Y. Les produits d'hydrolyse des blocs X et Y restent d'abord incorporés dans les agrégats hydrophobes formés lors de la préparation de l'hydrogel. Lorsque l'hydrogel est au contact d'un milieu aqueux, cette hydrolyse entraîne la libération progressive, par solubilisation dans le milieu aqueux, de polymères formés de chaînons G éventuellement liés à des blocs poly(hydroxyacide) suffisamment courts, de tels polymères étant solubles dans l'eau. Il en résulte une désagrégation progressive de l'hydrogel, qui se produit au contact du milieu aqueux, et qui peut s'accompagner de la libération dans ledit milieu d'une partie des macromolécules hydrophiles, des fragments courts provenant de la dégradation de X et Y, et des substances hydrophobes éventuellement encore présentes dans l'hydrogel.

L'invention concerne également un procédé de préparation d'un hydrogel tel que défini précédemment. Ce procédé est principalement caractérisée par le fait que l'on prépare une solution d'un copolymère X-G-Y tel que défini précédemment, dans un solvant organique miscible à l'eau, puis on met en contact cette solution avec une quantité suffisante d'eau pour former un hydrogel mou. Cet hydrogel mou est gonflé de liquide (solvant organique et eau). Pour éliminer le solvant organique, on peut ensuite laver l'hydrogel à l'eau et/ou le-soumettre à une dialyse contre de l'eau et/ou le lyophiliser.

Pour mettre en contact la solution organique avec l'eau, on peut soit verser la solution organique dans l'eau soit verser de l'eau dans la solution organique.

Pour préparer les hydrogels de l'invention, on peut notamment opérer de la façon suivante : on dissout le copolymère dans un solvant organique miscible à l'eau, puis on ajoute ensuite de l'eau de façon à obtenir la formation d'un gel mou.

L'addition d'eau peut être faite en une seule fois, ou de façon progressive, mais cependant pas trop lente pour éviter d'obtenir un hydrogel dispersé et manquant de cohésion. En fait la vitesse d'addition de l'eau (ou la vitesse d'addition du solvant organique dans l'eau quand on choisit cette autre méthode) qui convient dans chaque cas peut être déterminée au préalable par de simples expériences de routine.

Le solvant miscible à l'eau doit être un bon solvant des deux types de séquences X, Y d'une part et G d'autre part. Parmi les solvants utilisables on peut utiliser l'acétone, le dioxane, le tétrahydrofuranne, le diméthylformamide, le diméthylsulfoxyde.

On peut éliminer le solvant organique qui est présent, de même que l'eau, dans l'hydrogel obtenu, par lavages répétés à l'eau, ou par dialyse. La lyophilisation permet d'éliminer à la fois le solvant organique et l'eau.

Les hydrogels de l'invention sont notamment ceux qui sont préparés par le procédé qui vient d'être indiqué.

Pour incorporer une macromolécule hydrophile dans un hydrogel selon l'invention, on peut remplacer l'eau, dans le procédé qui vient d'être décrit, par une solution aqueuse de ladite macromolécule.

De même, pour incorporer une substance hydrophobe dans les hydrogels de l'invention, on peut remplacer, dans le procédé qui vient d'être décrit, le solvant organique soluble dans l'eau par une solution de la substance hydrophobe dans ledit solvant organique.

Pour sélectionner les copolymères capables de donner des hydrogels retenant une proportion suffisante d'eau, on peut par exemple opérer de la façon suivante : on dissout 200 mg du copolymère dans un volume pouvant aller de 0,4 à 0,8 cm³ de l'un des solvants organiques indiqués, par exemple l'acétone. On ajoute progressivement 4 cm³ d'eau de façon à former un hydrogel. On sélectionne par exemple les hydrogels qui, après élimination du liquide en excès, ont une masse au moins égale à 0,4 g.

Les hydrogels de l'invention peuvent libérer progressivement, notamment sous l'effet de la dégradation des chaînons X et Y (comme indiqué ci-dessus), les substances hydrophobes qui s'y trouvent solubilisées.

Par ailleurs, les hydrogels de l'invention présentent la particularité d'être biodégradables et biorésorbables. Des études de dégradation de ces copolymères triséquencés ont montré que les chaînes polyesters sont progressivement dégradées par hydrolyse, les produits finals étant les hydroxyacides (ou diacides et diols) correspondants qui sont biorésorbables.

Cette hydrolyse des chaînes polyesters, lorsqu'elle se poursuit, libère finalement le polymère hydrophile G formant le bloc central du copolymère triséquencé. On sait que de tels polymères de masse moléculaire relativement faible (inférieure à 10000), sont biorésorbables : ils sont éliminés par les reins.

Les polymères G mentionnés et les monomères constituant les chaînes X et Y des copolymères triséquencés qui forment les hydrogels de la présente demande sont des composés biodégradables ou biorésobables non toxiques bien tolérés par l'organisme.

Les hydrogels peuvent donc être utilisés notamment comme compositions pharmaceutiques permettant notamment de libérer progressivement les médicaments qu'elles renferment. De telles compositions s'avèrent particulièrement intéressantes dans le cas de principes actifs réputés insolubles dans l'eau. Les compositions de l'invention peuvent également être utilisées pour libérer progressivement des principes actifs hydrophiles, et en particulier des macromolécules hydrophiles, comme mentionné plus haut.

Parmi les substances hydrophobes qui peuvent être incorporées dans les hydrogels de l'invention on citera notamment : des hormones, en particulier des hormones stéroides (par exemple progestérone, estradiol, norgestrel, noréthistérone, testostérone, hydrocortisone, prednisolone, dexaméthasone), d'autres hormones (par exemple prostaglandines, insuline, etc.), des antibiotiques ou des antiparasitaires (par exemple griséofulvine, érythromycine, quinazoline et ses dérivés) des agents anticancéreux (par exemple nitrosourées, fluorouracile, azathioprine, doxorubicine, bléomycine, cis-platine, mitomycine) des anesthésiques ou des sédatifs (par exemple tétracaine, chloropromazine, diazépam, méthadone, naltrexone), ou d'autres principes actifs tels que la théophylline, la caféine, la quinidine, ou encore des peptides (notamment la vasopressine) .

On peut également incorporer dans les hydrogels de l'invention des immunogènes (notamment des protéines ou des conjugués protéine-antigène, par exemple des conjugués antigène-anatoxine tétanique, obtenus de façon connue en soi) et éventuellement des adjuvants. De tels gels sont utilisables comme agents vaccinants qui présentent l'avantage que, du fait de la libération progressive de l'immunogène, l'organisme se trouve plus longtemps au contact de celui-ci.

Les compositions pharmaceutiques de l'invention sont utilisables dans des procédés de traitement thérapeutique comprenant leur administration notamment par voie orale ou rectale. L'hydrogel ou le lyophilisat correspondant peut être encapsulé dans un matériau capable de se désagréger par fusion, ramollissement ou dissolution au contact du corps ou des fluides corporels. Ces compositions peuvent aussi être administrées sous la forme d'implants mous, notamment par injection.

Comme indiqué ci-dessus, les hydrogels selon l'invention sont des produits mous, qui peuvent avoir une forme propre, mais qui sont alors aisément déformables, par exemple sous la pression d'un doigt, comme les hydrogels bien connus formés par la gélatine ou le tapioca. Les hydrogels de l'invention et les compositions les contenant sont notamment des hydrogels suffisamment déformables pour pouvoir transiter à travers une aiguille creuse ayant par exemple 2 mm, ou même 1 mm, de diamètre intérieur.

Les hydrogels de l'invention peuvent aussi servir de support implantable et biorésorbable destiné à favoriser la régénération du parodonte selon la technique de régénération tissulaire et guidée (par exemple à la place des membranes décrites dans le document WO 93/24097).

Les hydrogels peuvent être stérilisés notamment par irradiation UV, mais il est généralement préférable de les préparer dans des conditions stériles.

On peut également incorporer dans les hydrogels de l'invention des substances colorantes, des substances aromatiques, des insecticides, etc.

Les hydrogels de l'invention peuvent être utilisés notamment comme supports pour la diffusion prolongée de parfums, d'arômes, d'insecticides.

Il est également possible d'incorporer dans les hydrogels de l'invention une ou plusieurs substances hydrophobes servant à moduler la stabilité des agrégats formant les microdomaines hydrophobes. On peut ajouter par exemple des oligomères obtenus à partir des mêmes monomères que ceux constituant les blocs X et Y de la formule (I).

L'invention a également pour objet un procédé pour le piégeage d'une substance hydrophobe dans un hydrogel, caractérisé par le fait que l'on prépare une solution d'une substance hydrophobe et d'un polymère triséquencé, tel que défini précédemment, dans un solvant organique miscible à l'eau qui est un solvant pour ladite substance hydrophobe et pour le copolymère, puis on met en contact ladite solution avec de l'eau pour obtenir un hydrogel qui est capable de retenir ladite substance hydrophobe. On peut opérer comme déjà décrit précédemment. Le solvant organique est choisi parmi ceux mentionnés ci-dessus.

L'invention concerne également un procédé de piégeage d'une macromolécule hydrophile dans un hydrogel, caractérisé par le fait que l'on prépare une solution contenant un copolymère triséquencé tel que défini ci-dessus, dans un solvant organique miscible à l'eau, puis que l'on met en contact ladite solution avec une solution aqueuse de ladite macromolécule, pour obtenir un hydrogel capable de retenir ladite macromolécule. On peut opérer comme déjà décrit précédemment.

L'invention s'étend bien entendu aux hydrogels, tels que définis ci-dessus, dans lesquels est solubilisée au moins une substance hydrophobe et/ou une macromolécule hydrophile.

La vitesse de dégradation des copolymères formant les hydrogels de l'invention dépend en partie du caractère plus ou moins amorphe ou cristallisé des chaînons X et Y. On sait qu'il est possible de contrôler en partie le caractère amorphe ou cristallin de tels chaînons. Par exemple, l'obtention de chaînons X et Y au départ de lactide racémique (au lieu d'un énantiomère) favorise l'obtention de chaînons amorphes ; par ailleurs, on sait que la cristallinité des chaînons X et Y augmente avec la longueur de ces chaînons ; voir notamment P. CERRAI et al., article cité ci-dessus. On peut donc régler en conséquence la vitesse de dégradation.

Les exemples suivants illustrent l'invention.

### EXEMPLES

### Exemple 1 : Préparation de copolymères

**1.1.** On mélange dans un ballon 0,4 mole de D,L-lactide (origine : Purac, Pays-Bas) avec 0,4 mole (en motifs répétitifs oxyéthylène) de PEG 2000 (Fluka, Suisse) et 37,6 mg (0,05 % du poids total des réactifs) de poudre de zinc (Merck, Allemagne). Le PEG 2000 a été préalablement purifié par dissolution dans le chloroforme et précipitation dans le diéthyléther à -10°C.
   On rappelle que la molécule de lactide comprend 2 mole de lactate. Le rapport du nombre de motifs lactate au nombre de motifs oxyéthylène dans le mélange de départ est donc égal à deux.
   Après dégazage sous vide du mélange réactionnel, on le chauffe à 140°C sous atmosphère d'argon jusqu'à ce que le mélange soit entièrement sous forme liquide. On réalise ensuite un vide de 10⁻² mm de mercure, on scelle le ballon et on le place dans un four à 140°C pendant 7 jours. Le ballon est ensuite ramené à la température ambiante, et son contenu est solubilisé dans l'acétone, puis soumis à une précipitation dans l'éthanol.
   Le produit obtenu est ensuite séché sous pression réduite.
   Le copolymère est analysé par RMN ¹H. En faisant le rapport de l'intégrale des protons CH des blocs PLA à 5,3-5,1 ppm à l'intégrale des protons (O-CH₂-CH₂) du bloc POE à 3,5 ppm, on a déterminé que le rapport molaire LA/OE dans le copolymère est égal à 2,1. On en déduit que la masse moléculaire du copolymère est de 8 800 environ.
**1.2.** De façon analogue à celle décrite à l'exemple 1.1, on a préparé au départ de PEG 2000 et de D,L-lactide (1,5 mole pour 1 mole (en motifs répétitifs oxyéthylène) de PEG) et de poudre de zinc (0,05 % du poids total des réactifs), un copolymère caractérisé par RMN ¹H par un rapport molaire LA/OE égal à 2,3, d'où une masse moléculaire de 9400 environ.
**1.3.** De façon analogue, au départ d'un mélange de 1,5 mole de D,L-lactide et de 1 mole (en motifs répétitifs oxyéthylène) de PEG 2000, en présence d'hydrure de calcium (Janssen, Belgique), à raison de deux moles d'hydrure par mole de PEG, on obtient un copolymère caractérisé par un rapport LA/OE égal à 3,2, d'où une masse molaire de 12 300 environ.
**1.4.** De façon analogue à celle décrite à l'exemple 1.3., on a préparé, au départ d'un mélange équimolaire de lactide et de PEG 2000, en présence d'hydrure de calcium, un copolymère ayant une masse moléculaire de 8 000 environ.
   Le mélange équimolaire mentionné ci-dessus fait référence, pour le PEG, à des moles de motifs répétitifs oxyéthylène.
**1.5.** De façon analogue à celle décrite à l'exemple 1.1., on a préparé, au départ de PEG 1000, de D,L-lactide, à raison d'une mole pour 1 mole (en motifs répétitifs oxyéthylène) de PEG et de poudre de zinc, un copolymère caractérisé en RMN ¹H par un rapport LA/OE égal à 3,4, d'où une masse molaire de 6400 environ.

### Exemple 2 : Obtention d'hydrogels

On dissout des échantillons de 200 mg de copolymère dans 0,4 cm³ d'acétone (ou dans 0,8 cm³ d'acétone) et on ajoute progressivement 4 cm3 d'eau. Les copolymères utilisés sont ceux des exemples 1.1. à 1.5. Dans chaque cas, on obtient un gel mou (hydrogel comportant une forte proportion d'eau).

En éliminant l'eau en excès et en la remplaçant par une nouvelle quantité d'eau, à plusieurs reprises, on élimine l'acétone.

On peut également éliminer l'acétone par dialyse ou par évaporation lente.

L'eau contenue dans l'hydrogel peut être éliminée par lyophilisation. Le lyophilisat, mis en présence d'eau, absorbe rapidement un poids d'eau au moins égal au poids de copolymère qu'il contient, et on obtient un hydrogel mou gonflé d'eau.

### Exemple 3 : Incorporation d'une substance hydrophobe dans un hydrogel

Dans 2 cm³ d'acétone, on dissout 1 g de copolymère de l'exemple 1.3 et 5 mg de colorant commercial Yellow OB.

Il s'agit d'un colorant très hydrophobe, insoluble dans l'eau. On ajoute progressivement de l'eau distillée (4 cm³). On obtient un gel occupant la presque totalité du volume de la solution acétonique de départ.

Le gel est coloré de façon homogène, sans apparition de cristaux de colorant.

En opérant de façon analogue, mais en l'absence de copolymère, le colorant cristallise lors de l'addition d'eau.

En lavant le gel à l'eau, comme décrit précédemment, pour éliminer l'acétone, le colorant reste présent dans le gel.

De façon analogue, on a préparé des hydrogels dans lesquels est incorporée de la progestérone. Même après plusieurs lavages du gel avec de l'eau, la plus grande partie de la progestérone reste piégée dans le gel. Lors des lavages du gel à l'eau, il n'y a pas formation d'un précipité de progestérone, contrairement à ce que l'on observe lorsque l'on ajoute de l'eau à une solution de progestérone dans l'acétone. Administré par injection sous-cutanée, le gel constitue un implant mou biorésorbable qui libère progressivement la progestérone.

### Exemple 4 : Incorporation d'une protéine

De façon analogue à celle décrite précédemment, on dissout 1 g de copolymère de l'exemple 1.3 dans 2 cm³ d'acétone et on ajoute progressivement de l'eau contenant en solution 10 g/1 d'albumine bovine.

On lave 4 fois à l'eau le gel obtenu.

On ajoute un volume de sérum physiologique égal au volume du gel.

On recherche la protéine dans le liquide en excès (par analyses UV effectuées toutes les 2 minutes) . La protéine ne devient détectable qu'au bout de 6 minutes et la libération se prolonge pendant plusieurs heures.

De façon analogue, on a incorporé, dans des hydrogels selon l'invention, d'autres protéines (notamment des immunoglobulines) et des conjugués protéine-antigènes dont la protéine était l'anatoxine tétanique et les antigènes étaient soit des polyosides ou fragments de polyosides de *Streptococcus pneumoniae,* soit des polyosides ou fragments de polyosides de *Salmonella typhi.*

## Revendications

1. Hydrogel à base de copolymère triséquencé et d'eau, contenant une quantité d'eau au moins égale, en poids, à celle dudit copolymère triséquencé, et par le fait que ledit copolymère répondant à la formule (I) :
X-G-Y (I)
dans laquelle
G est un bloc polymère linéaire hydrophile non hydroxylé contenant p motifs répétitifs, p étant un nombre pouvant varier de 10 à 150,
X et Y représentent chacun un bloc polyester contenant respectivement m et n motifs répétitifs,
le rapport (m + n)/p étant suffisamment élevé pour que ledit copolymère soit insoluble dans l'eau, caractérisé par le fait que le rapport (m + n)/p est choisi tel que l'addition d'eau à une solution du copolymère dans un solvant organique miscible à l'eau provoque la formation dudit hydrogel sous la forme d'un gel mou capable de transiter à travers une aiguille creuse ayant un diamètre interne de 2 mm.

2. Hydrogel selon la revendication 1, caractérisé par le fait que p est un nombre pouvant varier de 10 à 120, et en particulier de 15 à 100.

3. Hydrogel selon la revendication 1 ou 2, caractérisé par le fait que ledit rapport (m + n)/p est choisi pour que ledit gel puisse retenir une quantité d'eau au moins égale à deux fois le poids de copolymère qu'il contient.

4. Hydrogel selon l'une quelconque des revendications précédentes, caractérisé par le fait que ledit rapport (m + n)/p est un nombre compris entre 1 et 5 environ.

5. Hydrogel selon l'une quelconque des revendications précédentes, caractérisé par le fait que X et Y représentent : (i) des polymères qui dérivent des monomères choisis parmi l'acide lactique, l'acide glycolique, les monoesters d'acide malique, les lactides, le glycolide, la para-dioxanone, (ii) des copolymères que forment ces monomères entre eux, ou (iii) des polymères obtenus par polycondensation de diacides et de diols.

6. Hydrogel selon l'une quelconque des revendications précédentes, caractérisé par le fait que le bloc polymère représenté par G est choisi parmi les suivants : poly(éthylèneglycol), poly(vinylpyrrolidone) et polyacrylamide.

7. Hydrogel selon l'une quelconque des revendications précédentes, caractérisé par le fait qu'il renferme une substance hydrophobe.

8. Hydrogel selon la revendication 7, caractérisé par le fait que ladite substance hydrophobe est choisie parmi des médicaments, des substances colorantes, des substances aromatiques et des insecticides.

9. Hydrogel selon l'une quelconque des revendications 7 et 8, caractérisé par le fait que ladite substance hydrophobe est choisie parmi des hormones, des antibiotiques, des antiparasitaires, des agents anticancéreux, des anesthésiques, des sédatifs et des peptides.

10. Hydrogel selon l'une quelconque des revendications précédentes, caractérisé par le fait qu'il renferme une macromolécule hvdrosoluble.

11. Hydrogel selon l'une quelconque des revendications précédentes, capable de transiter à travers une aiguille creuse ayant un diamètre interne de 1mm.

12. Composition caractérisée par le fait qu'elle comprend comme support un hydrogel tel que défini dans l'une quelconque des revendications 1 à 6.

13. Composition caractérisée par le fait qu'elle comprend un hydrogel tel que défini dans l'une quelconque des revendications 7 à 11.

14. Composition pharmaceutique, caractérisée par le fait qu'elle comprend un hydrogel tel que défini dans l'une quelconque des revendications 1 à 7.

15. Composition selon la revendication 14, caractérisée par le fait qu'elle contient en outre un médicament comprenant une macromolécule hydrosoluble.

16. Procédé de préparation d'un hydrogel tel que défini dans l'une quelconque des revendications 1 à 11, caractérisé par le fait que l'on prépare une solution d'un copolymère X-G-Y, tel que défini dans l'une quelconque des revendications 1 à 6, dans un solvant organique miscible à l'eau, puis on met en contact cette solution avec une quantité suffisante d'eau pour former ledit hydrogel.

17. Procédé pour le piégeage d'une substance hydrophobe dans un hydrogel, caractérisé par le fait que l'on prépare une solution d'une substance hydrophobe et d'un polymère triséquencé tel que défini dans l'une quelconque des revendications 1 à 6, dans un solvant organique miscible à l'eau qui est un solvant pour ladite substance hydrophobe et pour le copolymère, puis on met en contact ladite solution avec de l'eau pour obtenir un hydrogel mou qui est capable de retenir ladite substance hydrophobe.

18. Procédé pour le piégeage d'une macromolécule hydrophile dans un hydrogel, caractérisé par le fait que l'on prépare une solution contenant un copolymère triséquencé tel que défini dans l'une quelconque des revendications 1 à 6, dans un solvant organique miscible à l'eau, puis on met en contact ladite solution avec une solution aqueuse de ladite macromolécule, pour obtenir un hydrogel mou capable de retenir ladite macromolécule.

19. Procédé selon l'une quelconque des revendications 16 à 18, caractérisé par le fait qu'en outre on lave ledit hydrogel à l'eau et/ou le soumet à une dialyse contre de l'eau.

20. Procédé d'obtention d'un lyophilisat, dans lequel on prépare un hydrogel selon le procédé de l'une quelconque des revendications 16 à 19, puis on hyophilise ledit hydrogel.

## Claims

1. Hydrogel based on triblock copolymer and on water comprising an amount of water at least equal, by weight, to that of the said triblock copolymer and the said copolymer corresponding to the formula (I):
X-G-Y (I)
in which G is a non-hydroxylated hydrophilic linear polymer block comprising p repeat units, p being a number which can vary from 10 to 150,
X and Y each represent a polyester block respectively comprising m and n repeat units,
the ratio (m + n)/p being sufficiently high for the said copolymer to be insoluble in water, characterized in that the ratio (m + n)/p is chosen such that the addition of water to a solution of the copolymer in a water-miscible organic solvent causes the formation of the said hydrogel in the form of a soft gel capable of passing through a hollow needle having an internal diameter of 2mm.

2. Hydrogel according to Claim 1, characterized in that p is a number which can vary from 10 to 120, and in particular from 15 to 100.

3. Hydrogel according to Claim 1 or 2, characterized in that the said ratio (m + n)/p is chosen so that the said gel can retain an amount of water at least equal to twice the weight of copolymer which it comprises.

4. Hydrogel according to any one of the preceding claims, characterized in that the said ratio (m + n)/p is a number between 1 and 5 approximately.

5. Hydrogel according to any one of the preceding claims, characterized in that X and Y represent: (i) polymers which derive from monomers chosen from lactic acid, glycolic acid, malic acid monoesters, lactides, glycolide or para-dioxanone, (ii) copolymers which these monomers form with one another, or (iii) polymers obtained by polycondensation of diacids and diols.

6. Hydrogel according to any one of the preceding claims, characterized in that the polymer block represented by G is chosen from the following: poly(ethylene glycol), poly(vinylpyrrolidone) and polyacrylamide.

7. Hydrogel according to any one of the preceding claims, characterized in that it comprises a hydrophobic substance.

8. Hydrogel according to Claim 7, characterized in that the said hydrophobic substance is chosen from medicaments, colorants, aromatic substances and insecticides.

9. Hydrogel according to either one of Claims 7 and 8, characterized in that the said hydrophobic substance is chosen from hormones, antibiotics, antiparasitics, anticancer agents, anaesthetics, sedatives and peptides.

10. Hydrogel according to any one of the preceding claims, characterized in that it comprises a water-soluble macromolecule.

11. Hydrogel according to any one of the preceding claims, capable of passing through a hollow needle having an internal diameter of 1mm.

12. Composition, characterized in that it comprises, as substrate, a hydrogel as defined in any one of Claims 1 to 6.

13. Composition, characterized in that it comprises a hydrogel as defined in any one of Claims 7 to 11.

14. Pharmaceutical composition, characterized in that it comprises a hydrogel as defined in any one of Claims 1 to 7.

15. Composition according to Claim 14, characterized in that it additionally comprises a medicament comprising a water-soluble macromolecule.

16. Process for the preparation of a hydrogel as defined in any one of Claims 1 to 11, characterized in that a solution of a copolymer X-G-Y, as defined in any one of Claims 1 to 6, is prepared in a water-miscible organic solvent and then this solution is brought into contact with a sufficient amount of water to form the said hydrogel.

17. Process for the trapping of a hydrophobic substance in a hydrogel, characterized in that a solution of a hydrophobic substance and of a triblock polymer as defined in any one of Claims 1 to 6 is prepared in a water-miscible organic solvent which is a solvent for the said hydrophobic substance and for the copolymer and then the said solution is brought into contact with water, in order to obtain a soft hydrogel which is capable of retaining the said hydrophobic substance.

18. Process for the trapping of a hydrophilic macromolecule in a hydrogel, characterized in that a solution comprising a triblock copolymer as defined in any one of Claims 1 to 6 is prepared in a water-miscible organic solvent and then the said solution is brought into contact with an aqueous solution of the said macromolecule, in order to obtain a soft hydrogel capable of retaining the said macromolecule.

19. Process according to any one of Claims 16 to 18, characterized in that, in addition, the said hydrogel is washed with water and/or subjected to dialysis against water.

20. Process for obtaining a lyophilisate, in which a hydrogel is prepared according to the process of any one of Claims 16 to 19 and then the said hydrogel is lyophilized.

## Patentansprüche

1. Hydrogel auf Basis eines trisequenziellen Copolymeren und Wasser, enthaltend eine Wassermenge, die mindestens in Gewicht derjenigen des trisequenziellen Copolymeren entspricht, und wobei das Copolymer der Formel (I) entspricht:
X-G-Y (I)
worin G ein lineares, hydrophiles, nicht hydroxyliertes Block polymer mit p wiederkehrenden Einheiten darstellt, wobei p ei ne Zahl ist, die zwischen 10 und 150 variieren kann,
X und Y jeweils für einen Blockpolyester stehen, enthaltend jeweils m und n wiederkehrende Einheiten,
worin das Verhältnis (m + n)/p ausreichend hoch ist, so dass das Copolymer in Wasser unlöslich ist, dadurch gekennzeichnet, dass das Verhältnis (m + n)/p so gewählt ist, dass die Addition von Wasser zu einer Lösung des Copolymeren in einem mit Wasser mischbaren organischen Lösungsmittel die Bildung des Hydrogels in Form eines nachgiebigen Gels bewirkt, das in der Lage ist, durch eine Hohlnadel mit einem Innendurchmesser von 2 mm hindurchzutreten.

2. Hydrogel gemäß Anspruch 1, dadurch gekennzeichnet, dass p eine Zahl ist, die zwischen 10 und 120 und insbesondere zwischen 15 und 100 schwanken kann.

3. Hydrogel gemäß Anspruch 1 oder 2, dadurch gekennzeichnet, dass das Verhältnis (m + n)/p so gewählt ist, dass das Gel eine Wassermenge zurückhalten kann, die mindestens dem Zweifachen des Gewichts des in ihm enthaltenen Copolymeren entspricht.

4. Hydrogel gemäß einem der vorstehenden Ansprüche, dadurch gekennzeichnet, dass das Verhältnis (m + n)/p eine Zahl zwischen 1 und etwa 5 darstellt.

5. Hydrogel gemäß einem der vorstehenden Ansprüche, dadurch gekennzeichnet, dass X und Y darstellen: (i) Polymere, die von Monomeren stammen, ausgewählt unter Milchsäure, Glykolsäure, den Monoestern der Äpfelsäure, den Lactiden, dem Glycolid, dem Paradioxanon, (ii) den Copolymeren, die diese Monomere untereinander bilden, oder (iii) den Polymeren, die durch Polykondensation von Disäuren und Diolen erhalten werden.

6. Hydrogel gemäß einem der vorstehenden Ansprüche, dadurch gekennzeichnet, dass das durch G dargestellte Blockpolymer ausgewählt ist unter den folgenden: Poly(ethylenglykol), Poly(vinylpyrrolidon) und Polyacrylamid.

7. Hydrogel gemäß einem der vorstehenden Ansprüche, dadurch gekennzeichnet, dass es eine hydrophobe Substanz umfasst.

8. Hydrogel gemäß Anspruch 7, dadurch gekennzeichnet, dass die hydrophobe Substanz ausgewählt ist unter Medikamenten, farbgebenden Substanzen, aromatischen Substanzen und Insektiziden.

9. Hydrogel gemäß einem der Ansprüche 7 und 8, dadurch ge kennzeichnet, dass die hydrophobe Substanz ausgewählt ist unter Hormonen, Antibiotika, Mitteln gegen Parasiten, Mitteln gegen Krebs, Anästhetika, Sedativa und Peptiden.

10. Hydrogel gemäß einem der vorstehenden Ansprüche, dadurch gekennzeichnet, dass es ein wasserlösliches Makromolekül enthält.

11. Hydrogel gemäß einem der vorstehenden Ansprüche, das in der Lage ist, durch eine Hohlnadel mit einem Innendurchmesser von 1 mm hindurchzutreten.

12. Zubereitung, dadurch gekennzeichnet, dass sie als Träger ein Hydrogel gemäß einem der Ansprüche 1 bis 6 enthält.

13. Zubereitung, dadurch gekennzeichnet, dass sie ein Hydrogel gemäß einem der Ansprüche 7 bis 11 enthält.

14. Pharmazeutische Zubereitung, dadurch gekennzeichnet, dass sie ein Hydrogel gemäß einem der Ansprüche 1 bis 7 enthält.

15. Zubereitung gemäß Anspruch 14, dadurch gekennzeichnet, dass sie zusätzlich ein Medikament enthält, umfassend ein wasserlösliches Makromolekül.

16. Verfahren zur Herstellung eines Hydrogels gemäß einem der Ansprüche 1 bis 11, dadurch gekennzeichnet, dass man eine Lösung eines Copolymeren X-G-Y, wie in einem der Ansprüche 1 bis 6 definiert, in einem mit Wasser mischbaren organischen Lösungsmittel herstellt, anschließend diese Lösung mit einer zur Bildung des Hydrogels ausreichenden Menge an Wasser in Kontakt bringt.

17. Verfahren zur Verkapselung einer hydrophoben Substanz in einem Hydrogel, dadurch gekennzeichnet, dass man eine Lösung einer hydrophoben Substanz und eines trisequenziellen Polymeren, wie in einem der Ansprüche 1 bis 6 definiert, in einem mit Wasser mischbaren organischen Lösungsmittel herstellt, das ein Lösungsmittel für die hydrophobe Substanz und das Copolymer darstellt, anschließend die Lösung mit Wasser zum Erhalt eines nachgiebigen Hydrogels in Kontakt bringt, das die hydrophobe Substanz zurückhalten kann.

18. Verfahren zur Verkapselung eines hydrophilen Makromole küls in einem Hydrogel, dadurch gekennzeichnet, dass man eine Lösung, die ein trisequenzielles Copolymer enthält, das gemäß einem der Ansprüche 1 bis 6 definiert ist, in einem mit Wasser mischbaren organischen Lösungsmittel herstellt, anschließend die Lösung mit einer wässrigen Lösung des Makromoleküls in Kontakt bringt, um ein nachgiebiges Hydrogel zu erhalten, das das Makromolekül zurückhalten kann.

19. Verfahren gemäß einem der Ansprüche 16 bis 18, dadurch gekennzeichnet, dass man das Hydrogel zusätzlich mit Wasser wäscht und/oder dieses einer Dialyse gegen Wasser unterwirft.

20. Verfahren zum Erhalt eines Lyophilisats, bei dem man ein Hydrogel gemäß einem Verfahren nach einem der Ansprüche 16 bis 19 herstellt, anschließend das Hydrogel lyophilisiert.
